(19) Europäisches Patentamt
European Patent Office
Office européen des brevets

(11) **EP 0 930 933 B1**

(12) **FASCICULE DE BREVET EUROPEEN**

(45) Date de publication et mention
de la délivrance du brevet:
**16.04.2003 Bulletin 2003/16**

(51) Int Cl.⁷: **B01F 17/00**

(21) Numéro de dépôt: **98941547.6**

(86) Numéro de dépôt international:
**PCT/FR98/01748**

(22) Date de dépôt: **05.08.1998**

(87) Numéro de publication internationale:
**WO 99/007463 (18.02.1999 Gazette 1999/07)**

(54) **PROCEDE DE LIBERATION D'UN PRINCIPE ACTIF CONTENU DANS UNE EMULSION MULTIPLE**

VERFAHREN ZUR FREISETZUNG EINES IN EINER MULTIPLEN EMULSION ENTHALTENEN
AKTIVBESTANDTEILS

METHOD FOR RELEASING AN ACTIVE PRINCIPLE CONTAINED IN A MULTIPLE EMULSION

(84) Etats contractants désignés:
**AT BE CH DE ES FR GB GR IE IT LI LU NL PT SE**

(30) Priorité: **07.08.1997 FR 9710154**

(43) Date de publication de la demande:
**28.07.1999 Bulletin 1999/30**

(73) Titulaire: **CENTRE NATIONAL DE
LA RECHERCHE SCIENTIFIQUE (CNRS)
75016 Paris (FR)**

(72) Inventeurs:
• **BIBETTE, Jérôme
F-33000 Bordeaux (FR)**

• **FICHEUX Marie-Françoise
F-33170 Gradignan (FR)**
• **LEAL CALDERON, Fernando
F-33650 La Brède (FR)**
• **BONNAKDAR, Lida
F-33400 Talence (FR)**

(74) Mandataire: **Bernasconi, Jean Raymond et al
c/o Cabinet Lavoix,
2, Place d'Estienne d'Orves
75441 Paris Cedex 09 (FR)**

(56) Documents cités:
**EP-A- 0 717 978        DE-A- 19 509 301**

**Description**

La présente invention a pour objet un procédé pour libérer un principe actif contenu dans une émulsion multiple.

Par définition, une émulsion consiste en une dispersion de deux phases non miscibles entre elles, comme généralement un mélange d'une phase aqueuse et d'une phase huileuse. Une émulsion est dite directe, lorsqu'elle comprend des gouttelettes d'huile dispersées dans une phase aqueuse et inverse, lorsqu'il s'agit de gouttelettes d'eau dispersées dans une phase huileuse.

Les émulsions sont classiquement obtenues en cisaillant l'une des phases à l'intérieur de l'autre phase, en présence d'au moins un agent de surface de type tensioactifs, polymères...

Ces agents de surface sont en fait choisis en fonction de la nature de l'émulsion envisagée. Dans le cas d'une émulsion directe, sont privilégiés les agents de surface possédant une balance hydrophile/lipophile (HLB) supérieure à 14. En revanche, des agents de surface d'HLB inférieure à 7, sont de préférence mis en oeuvre dans des émulsions inverses.

Le terme "HLB" (Hydrophilic Lipophilic Balance) désigne le rapport de l'hydrophilie des groupements polaires des molécules de tensioactifs à l'hydrophobie de la partie lipophile de ces mêmes molécules ; il s'agit d'un terme couramment utilisé dans le domaine des agents de surface (voir le Traité 'Techniques de l'Ingénieur", chapitre A7610 : "Les agents de surface").

Les émulsions incorporant ces agents de surface, demeurent métastables dans un délai suffisamment long pour permettre leur valorisation dans de nombreux domaines d'application comme par exemple les industries cosmétique, de revêtement, alimentaire et pharmaceutique.

Dans le cas particulier des émulsions multiples, on a une superposition d'au moins deux émulsions. il s'agit par exemple d'une dispersion, dans une phase aqueuse, de globules huileux dans lesquels sont dispersées de minuscules gouttelettes d'eau. Chacune de ces deux émulsions est bien entendu stabilisée grâce à l'incorporation, dans sa phase continue, d'un agent de surface tel que défini précédemment.

La présente invention vise précisément à tirer profit de ce type d'émulsion multiple, eau dans huile dans eau, pour véhiculer au moins un principe actif hydrophile et en permettre le relargage de manière contrôlée.

De part sa structure triphasique, une émulsion multiple, eau dans huile dans eau, s'avère avantageusement propice à l'encapsulation, au niveau de ses gouttelettes d'eau internes, de principes actifs hydrophiles. Les globules de la phase huileuse, dans lesquels sont émulsionnées lesdites gouttelettes d'eau confèrent un excellent barrage protecteur audits principes actifs, vis-à-vis du milieu extérieur. On peut ainsi envisager de véhiculer ces principes actifs via l'émulsion multiple.

La présente invention a précisément pour objet de proposer un procédé permettant de contrôler la libération de ce principe actif, encapsulé dans une émulsion multiple.

Au sens de la présente invention on entend définir par émulsion multiple, une émulsion eau dans huile dans eau, comprenant une émulsion inverse, Ei avec une phase aqueuse A1, en dispersion sous forme de gouttelettes d'émulsion directe Ed, dans une phase continue aqueuse A2, avec les deux émulsions Ed et Ei contenant, au niveau de leurs phases continues respectives, au moins un agent de surface en quantité suffisante pour les maintenir sous des formes stabilisées et différenciées au sein de ladite émulsion multiple. Le principe actif hydrophile contenu dans l'émulsion multiple considérée selon l'invention, est présent au niveau de la phase aqueuse A1.

La démarche retenue pour libérer ce principe actif de l'émulsion multiple tire en fait partie des phénomènes de coalescence, susceptibles de se manifester au sein de celle-ci.

Par définition, une coalescence est une rupture d'un film fin, établi entre deux gouttes adjacentes. Dans le cas d'une émulsion multiple, ce type de coalescence peut se manifester à deux niveaux, le premier, entre les gouttelettes d'eau internes, présentes dans un globule huileux et le second, entre l'interface d'un globule huileux et certaines de ses gouttelettes d'eau internes.

De manière surprenante, il s'avère possible de contrôler ces phénomènes de coalescence c'est-à-dire de s'y opposer ou au contraire de les induire grâce à la mise en oeuvre dans ladite émulsion, de concentrations spécifiques en agents de surface.

Les études, réalisées dans le cadre de la présente invention, ont ainsi permis de mettre en évidence que le phénomène de coalescence déterminant plus particulièrement une instabilité de l'émulsion multiple, propice à la libération du principe actif, contenu dans les gouttelettes d'eau internes A1, est celui s'effectuant à l'interface d'un globule huileux et de certaines de ses gouttelettes d'eau internes A1. Il s'en suit un transfert du principe actif, contenu initialement dans ces gouttelettes d'eau A1, vers la phase aqueuse A2. Le principe actif se trouve alors en contact avec le milieu extérieur, contenant à l'origine ladite émulsion multiple, et peut donc exercer son activité.

Avantageusement, il s'avère possible de s'opposer ou au contraire d'induire la manifestation de ce phénomène de coalescence, en intervenant au niveau de la concentration en agent de surface dans la phase continue aqueuse A2 de l'émulsion Ed. Il existe un seuil de concentration critique au delà duquel on peut déclencher la fuite du contenu des gouttelettes d'eau internes A1 vers le milieu extérieur.

En conséquence, la présente invention propose un procédé pour libérer de manière contrôlée un principe actif contenu dans une émulsion multiple de type eau dans huile dans eau, ladite émulsion multiple comprenant une émulsion inverse Ei avec une phase aqueuse A1 qui contient au moins un principe actif hydrophile, ladite émulsion Ei étant dispersée sous forme de gouttelettes d'émulsion directe Ed, dans une phase continue aqueuse A2, avec les deux émulsions Ed et Ei stabilisées par au moins un agent de surface, présent au niveau de leurs phases continues respectives, l'agent de surface contenu dans la phase aqueuse A2 de l'émulsion Ed étant un agent de surface hydrosoluble possédant une HLB supérieure à 14, présent à une concentration inférieure à la concentration dite concentration-seuil critique, au-delà de laquelle on induit une déstabilisation de l'émulsion multiple,
caractérisé en ce que ladite émulsion multiple est mise en présence d'une quantité suffisante d'un agent de surface pour que la concentration en agent de surface dans la phase A2 devienne supérieure à la concentration-seuil critique, de manière à transformer l'émulsion multiple en une émulsion directe et à induire la libération du principe actif, contenu dans la phase aqueuse A1 de l'émulsion Ei, dans la phase aqueuse A2.

Au sens de l'invention on entend définir par une concentration-seuil critique, la valeur de concentration en agent surface au-delà de laquelle on induit une déstabilisation de l'émulsion multiple avec pour effet la libération du principe actif.

Si la concentration en tensioactif hydrophile présent dans la phase aqueuse A2, est inférieure à cette concentration-seuil, alors on n'observe pas de coalescence pendant une période de l'ordre de plusieurs mois.

A l'inverse, si la concentration en tensioactif hydrophile présent dans la phase aqueuse A2, est supérieure ou égale à cette concentration-seuil, alors la coalescence et donc le relargage s'effectuent sur une échelle de temps variant de quelques jours à quelques minutes. Plus cette concentration est élevée, plus le temps de relargage est court.

La libération du principe actif sera considérée achevée lorsque la quasi-totalité des gouttelettes de la phase aqueuse A1 auront été relarguées dans la phase aqueuse externe A2. Cette libération sera donc appréciée rapide ou lente, selon le temps écoulé pour l'exécution de ce relargage.

Avantageusement, il s'avère ainsi possible de contrôler la libération du principe actif sur une durée plus ou moins longue en ajustant l'écart de concentration entre la concentration-seuil critique et la concentration finale, obtenue par ajout d'agent de surface.

En fait, il a été observé que cette concentration-seuil pouvait être exprimée par rapport à la concentration micellaire critique, CMC, de l'agent de surface considéré.

La concentration micellaire critique est définie comme la concentration au-delà de laquelle les molécules tensioactives s'associent pour former des amas sphériques appelés micelles (voir par exemple "Galenica 5, agents de surfaces et émulsions", vol. 5.1, page 101, éditeur : Techniques et Documentation (Lavoisier)).

Toutefois, la valeur de cette concentration-seuil, exprimée par rapport à la CMC, varie également selon la valeur HLB de l'agent de surface.

C'est ainsi que pour un certain domaine de valeurs HLB, les agents de surface correspondants devront être présents, dans la phase aqueuse A2, à une concentration inférieure à leur concentration micellaire critique, si l'on veut éviter la libération du principe actif.

En revanche, pour un autre domaine de valeurs HLB, les agents de surface correspondants pourront être présents jusqu'à une concentration très supérieure à leur CMC, sans que soit observée cette libération du principe actif.

En règle générale, pour des agents de surface possédant une HLB de l'ordre de 40, c'est-à-dire très hydrophiles, la concentration seuil est comprise entre 1 et 20 CMC.

Dans le cas des agents de surface possédant une HLB comprise entre environ 12 et 20, cette concentration seuil est supérieure à 100 CMC.

De même, le diamètre des gouttelettes d'eau internes A1, le diamètre des gouttelettes d'émulsion Ed, la nature chimique du principe actif présent dans la phase aqueuse A1 ainsi que la quantité et le type de tensioactif présent dans la phase huileuse de l'émulsion Ei affectent la valeur de cette concentration-seuil critique.

L'ensemble de ces paramètres sont donc à considérer pour apprécier la concentration-seuil critique pour un agent de surface spécifique, présent dans la phase continue aqueuse A2 de l'émulsion Ed.

L'appréciation de cette concentration-seuil critique, pour un agent de surface d'HLB donnée, peut être facilement effectuée à partir d'essais préliminaires selon le protocole décrit dans l'exemple 1 ci-après.

Cette appréciation peut par exemple être réalisée selon le protocole consistant à :

- préparer une émulsion multiple, incorporant le principe actif dans sa phase aqueuse interne, et comprenant un agent de surface dans la phase aqueuse externe A2 en quantité suffisante pour stabiliser ladite émulsion,
- ajouter dans la phase aqueuse A2 de ladite émulsion des quantités croissantes dudit agent de surface,
- doser, à l'issue de chaque ajout dudit agent de surface, la concentration en principe actif ayant ou non été relargué dans la phase aqueuse externe et
- relever la concentration en agent de surface à partir de laquelle est observée une accélération significative de la cinétique de relargage.

Généralement on relève la concentration en agent de surface à partir de laquelle 90 % du principe actif initialement présent au sein de la phase interne sont retrouvés dans la phase aqueuse externe en un laps de temps d'environ 10 heures.

En ce qui concerne la technique de dosage retenue pour estimer la concentration du principe actif relargué, elle varie bien entendu en fonction de la nature de ce principe actif. Il peut s'agir d'une mesure conductimétrique ou potentiométrique lorsque le principe actif est une espèce ionique ou encore une technique par spectroscopie de fluorescence si le principe actif est fluorescent.

Bien entendu l'homme de l'art est à même de sélectionner la technique de dosage adéquate.

Avantageusement, il s'avère donc possible d'induire la libération du principe actif, contenu dans l'émulsion revendiquée, en mettant en présence l'émulsion directe Ed, avec un agent tel qu'il provoque, de part sa présence et éventuellement sa concentration, un transfert des gouttelettes d'eau internes A1 vers la phase aqueuse A2, transformant ainsi l'émulsion multiple en une simple émulsion directe avec libération du principe actif dans le milieu extérieur.

Selon un mode privilégié de l'invention, cet agent est de préférence un agent de surface qui est identique à l'agent de surface, présent dans la phase aqueuse A2 de l'émulsion Ed et tel que défini ci-après. De part sa présence, il déplace la concentration initiale de cet agent de surface au delà de sa concentration-seuil critique et déclenche ainsi le phénomène de coalescence propice à la libération du principe actif.

Toutefois, on peut également envisager que cette libération soit induite par un agent de nature différente de celle de l'agent de surface, mis en oeuvre dans la phase aqueuse A2. Il peut ainsi s'agir d'un autre agent de surface ou encore d'un composé déjà présent dans le milieu extérieur où l'on envisage de libérer le principe actif.

Cet agent peut notamment être un polymère de type polyvinyl pyrrolidone, polyéthylène glycol ou encore un hydrocolloïde comme la gomme xanthane, le guar, la carraghuénane ainsi que leurs dérivés.

De préférence, l'agent de surface présent au niveau de la phase aqueuse A2 est un agent de surface hydrosoluble possédant une HLB supérieure à 14.

A titre illustratif de ce type d'agents hydrosolubles, on peut notamment citer les lécithines hydrosolubles, esters de sucrose, esters d'acides gras (dont les "Tweens"), alkylamides polyoxyéthylénés, triglycérides sulfates, alkyles sulfates (dont le dodécyle sulfate de sodium SDS), alkyles éther sulfates, alkyles sulfonates, sels d'alkylamines (dont le Bromure de Tétradécyl Triméthylammonium TTAB), amines grasses, lipoaminoacides (dont les sérums d'albumine bovine ou humaine, la bétalactoglobuline, la caséine), alkylbétaines, alkylpolyglycol éthers, copolymères d'oxydes d'alkylènes, polyesters modifiés et tensioactifs polymériques siliconés.

En ce qui concerne l'agent de surface présent dans la phase huileuse continue de l'émulsion Ei, il s'agit de préférence d'un agent de surface liposoluble d'HLB inférieure à 7.

Les agents de surface liposolubles, susceptibles d'être mis en oeuvre dans l'émulsion selon l'invention, peuvent être choisis parmi les lécithines liposolubles, les esters de sorbitanne et d'acides gras (dont les "Span"), polyalkylènes dipolyhydroxystéarates, acides gras, monoglycérides, esters de polyglycérol, polyricinoléate de polyglycérol et esters d'acide lactique et tartrique.

La phase continue de l'émulsion Ei est une phase huileuse composée de préférence d'au moins une huile choisie parmi les huiles végétales, animales ou minérales.

L'émulsion inverse Ei comprend de préférence environ 50 % à 99 % en volume de cette phase continue pour 1 % à 50 % de phase aqueuse A1.

Quant à l'émulsion directe Ed, elle comprend de préférence de 50 % à 99 % en volume de phase aqueuse A2 pour 1 % à 50 % de cette émulsion inverse Ei.

En ce qui concerne le principe actif hydrophile, présent dans l'émulsion revendiquée, il peut s'agit d'un composé actif dans l'un des domaines suivants à savoir pharmaceutique, cosmétique, phytosanitaire, alimentaires et/ou des revêtements de type peinture ou routier par exemple.

Il peut ainsi être choisi parmi les vitamines (E, C), enzymes, insuline, agents antalgiques, antimitotiques, anti-inflammatoires ou anti-glaucomateux, vaccins, agents anti-cancéreux, antagonistes narcotiques, agents de détoxication (salicylates, barbiturates), agents dépilatoires, agents correcteurs ou masqueurs de goût, sels hydrosolubles, agents rupteurs (émulsions de bitume) acides, bases, vinaigre, glucose, colorants, conservateurs ou leurs mélanges.

Bien entendu, la concentration en ce principe actif de la phase aqueuse A1 est à définir dans chaque cas particulier par l'homme de l'art en fonction de l'efficacité attendue.

L'invention est en particulier utile pour accélérer la rupture des émulsions du bitume lors de leur étalement sur les chaussées. Dans cette application particulière, on incorpore un agent rupteur, généralement un sel ou une solution à pH basique dans une émulsion multiple et on en provoque son relargage par mise en contact de ladite émulsion avec l'émulsion de bitume. Dans ce cas particulier, c'est la présence de l'agent de surface présent dans l'émulsion de bitume qui initie le relargage de cet agent rupteur. Il s'en suit donc une accélération de la rupture du bitume, induite précisément par cet agent rupteur.

La présente invention vise en particulier l'application du procédé revendiqué au relargage d'un agent rupteur dans le domaine des revêtements routiers et plus particulièrement dans le cadre de l'application d'un revêtement à base de bitume.

Dans un autre domaine d'activité comme celui de la pharmacie, l'invention permet également le temps de relargage des temps de principe actif.

Ceci est avantageux sur deux points, il s'en suit une meilleure assimilation du principe actif par l'organisme traité et une optimisation de l'efficacité du principe actif.

Les exemples et figures présentés ci-après sont soumis à titre illustratif et non limitatif de la présente invention.

FIGURE 1 : Photographie microscopique d'une émulsion multiple après 2 mois de stockage à température ambiante.
FIGURE 2 : Détermination de la concentration seuil critique d'un agent de surface dans une émulsion multiple.
FIGURES 3 : Evolution structurale d'une émulsion multiple. Photographie microscopique de l'émulsion fraîchement préparée (3a) et après 2 jours de stockage (3b).
FIGURES 4 : Evolution structurale au cours du temps d'une émulsion multiple. Photographie au temps zéro (4a), à 5 heures (4b) et après 13 jours de stockage (4c).
FIGURE 5 : Courbe de cinétique de relargage.

## EXEMPLE 1

Préparation d'une émulsion multiple stabilisée par un agent de surface d'HLB égal à 40, dans la phase aqueuse A2.

Dans un premier temps, on prépare une émulsion inverse, eau dans dodécane, monodispersée et stabilisée par du Span 80®, (monooléate de sorbitan de la société Sigma). Cette émulsion est préparée en introduisant lentement la phase aqueuse dispersée (80 % en volume) sous faible cisaillement (de l'ordre de 1 000 s$^{-1}$) dans une phase continue constituée d'un mélange dodécane/Span 80 dans un rapport pondéral 1/1. On ajoute du chlorure de sodium NaCl (0,1 M) destiné à simuler la présence du principe actif dans la phase aqueuse A1. Avantageusement, il a été démontré que la présence de ce sel renforce la stabilité de l'émulsion inverse Ei (Aronson M.P., Petko M.F., J. Colloid Interface Sci. 1993, 159, 134). L'émulsion polydispersée initiale est transformée par une technique de cristallisation fractionnée (Bibette J., J. Colloid Interface Sci. 1991, **147**, 474) en une émulsion monodispersée.

Le diamètre des gouttes est d'environ 0,3 µm.

La stabilité de cette émulsion a été éprouvée au cours du temps. De même, l'addition d'un agent de surface jusqu'à une concentration de 2 % en masse, à une température de 20°C, dans l'émulsion, diluée dans du dodécane à raison de 10 % en volume, n'induit aucune séparation de phase ou de phénomène d'agrégation.

Il a été ensuite préparé plusieurs émulsions multiples à partir de cette émulsion inverse en la mélangeant à une solution aqueuse contenant du sulfate de dodécyle de sodium (SDS, HLB = 40) à diverses concentrations, inférieures et supérieures à sa concentration micellaire critique qui est de 8.10$^{-3}$ moles/l.

L'essai consiste à mettre en présence avec précaution (sur une surface de 1 cm$^2$) des volumes équivalents (1 cm$^3$) de l'émulsion inverse et d'eau contenant des quantités variables en agent de surface SDS et une concentration en AgNO$_3$, à une concentration de 10$^{-3}$ moles/l. On détecte le transfert de l'eau, contenue dans les gouttelettes inverses, vers la phase aqueuse macroscopique, par l'observation d'un précipité d'AgCl qui se forme à l'interface macroscopique, lorsque le contenu interne transfert vers la phase externe. En effet, lorsque les gouttelettes d'eau se déplacent via un phénomène de coalescence de l'émulsion inverse supérieure à la phase aqueuse inférieure, la phase supérieure devient progressivement transparente alors que la phase inférieure devient progressivement laiteuse. On observe que pour une concentration inférieure à environ 10 CMC en SDS, les gouttelettes d'eau ne transfèrent pas vers la seconde phase même pour un temps s'écoulant sur plusieurs jours.

Au-dessus de cette concentration limite, les gouttelettes d'eau transfèrent rapidement (en moins de 48 heures) à l'intérieur de la seconde phase.

Afin de réduire le délai de diffusion à travers la phase supérieure, le même essai a été répété sous faible centrifugation. En appliquant une accélération de l'ordre de 10$^4$ g (g étant l'accélération de gravité) pendant 15 minutes, les gouttes de l'émulsion inverse soit se concentrent au niveau de l'interface eau-huile ou transfèrent vers la phase aqueuse inférieure. Le transfert via le phénomène de coalescence se réalise pour la même concentration à l'intérieur de la phase aqueuse inférieure c'est-à-dire 10 CMC en SDS.

## EXEMPLE 2

Détermination de la concentration seuil critique d'un tensioactif dans une émulsion multiple.

Le but de cette étude est de suivre de manière quantitative la libération d'un sel contenu dans une émulsion double.

I. Obtention d'une émulsion double monodisperse.

L'émulsion double est préparée en deux étapes.

1ère étape

On prépare une émulsion inverse, eau dans dodécane, monodisperse et stabilisée par du span 80.

On introduit la phase aqueuse (80 % en volume) sous faible cisaillement (de l'odre de 1000 s$^{-1}$) dans une phase continue constituée d'un mélange dodécane/span (1:1 massique). La phase aqueuse contient du chlorure de potassium à 1 mole par litre. Ce sel joue le rôle de la substance active devant être relarguée. L'émulsion polydisperse initiale est transformée par une technique de cristallisation fractionnée de façon à obtenir une émulsion monodispersée (Bibette J., J. Colloid Interface Sci., 1991, **147,** 474).

Les caractéristiques de l'émulsion inverse obtenue après dilution sont :

■    Fraction volumique d'eau salée : $\phi_{iv}$ = 10 %.
■    Concentration massique en span 80 dans le dodécane = 2 % m.
■    Diamètre des gouttelettes : $\sigma i$ = 0,3 $\mu$m.

2ème étape

L'émulsion inverse précédente est à son tour émulsionnée dans une phase aqueuse contenant un tensioactif hydrophile (SDS). On obtient ainsi une émulsion double constituée de globules huileux contenant des gouttelettes d'eau issues de la première étape.

On fabrique l'émulsion double à l'aide d'un microfluidiseur (Jet homogenizer de chez LABPLANT). Cette méthode consiste à mettre en contact, sous très haute pression, la phase dispersée et la phase continue, puis à éjecter le mélange par un trou de faible diamètre (0,1 mm).

Les caractéristiques de l'émulsion double obtenue sont :

■    Fraction volumique d'émulsion inverse : $\phi_{iv}$ = 20 %.
■    Concentration en SDS dans la phase aqueuse externe : $C_{SDS}$ = CMC/10.
■    Taille moyenne des globules = 4 $\mu$m.

II. Etude de la libération du sel.

II.1. Principe du dosage potentiométrique.

La concentration du KCl, initialement incorporé dans la phase aqueuse interne de l'émulsion double est mesurée dans la phase auqueuse externe par potentiométrie.

Le dosage potentiométrique repose sur la mesure d'une différence de potentiel entre une électrode indicatrice (Ag/AgCl) et une électrode de référence (électrode au sulfate mercureux) plongées dans la solution colloïdale contenant l'électrolyte à doser (ion Cl⁻). La concentration de l'ion Cl⁻ est dans ce cas directement reliée au potentiel $\Delta E$ de l'électrode Ag/AgCl par une relation du type :

$$\Delta E = \beta + \alpha \log Cq \text{ (relation de NERNST).}$$

On peut alors, par l'intermédiaire d'une droite d'étalonnage, connaître à tout instant la concentration en ion Cl⁻ libérée dans la phase aqueuse externe.

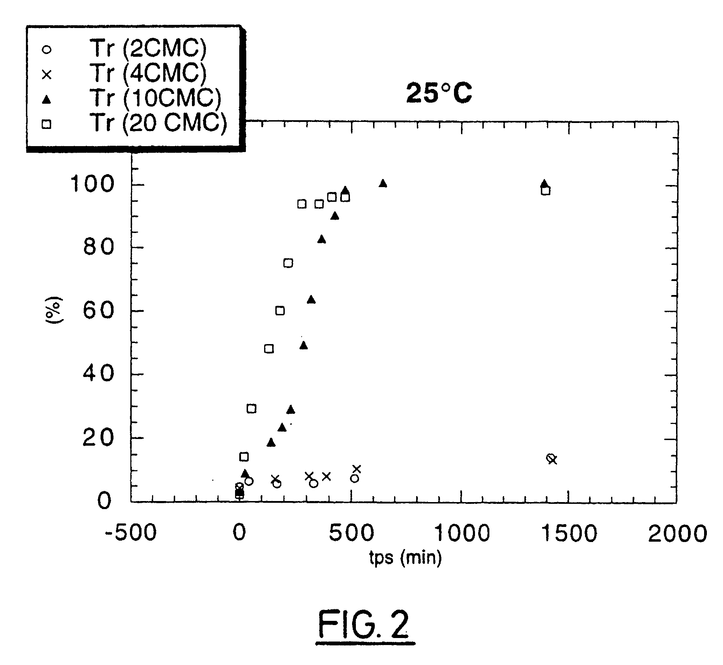

Les résultats obtenus sont présentés sous forme de graphe dans la figure 2.

L'abscisse de la courbe représente le temps exprimé en minutes et l'ordonnée représente le pourcentage de sel relargué dans la phase aqueuse externe.

Les cinétiques obtenues mettent en évidence, avec les observations microscopiques, deux scénarios de libération du sel.

Dans l'un des cas, pour une concentration en SDS supérieure ou égale à 10 CMC, on constate que le relargage se fait rapidement (temps correspondant à une libération totale du sel inférieur à 500 minutes) et que la libération est d'autant plus rapide que la concentration en SDS est importante. On observe dans ce cas, par microscopie optique, une transformation de l'émulsion double en émulsion simple (absence de gouttelettes dans les globules). On en déduit qu'il s'agit d'une libération par coalescence.

A l'inverse, lorsque la concentration en SDS est inférieure à la concentration seuil de 10 CMC, on note que la libération du sel dans la phase aqueuse externe est très lente (exemples comparatifs). Cette libération a lieu par diffusion passive et non par coalescence car l'émulsion conserve son caractère double.

**EXEMPLE 3**

Influence de la concentration en agent de surface dans l'émulsion directe sur la stabilité de l'émulsion multiple.

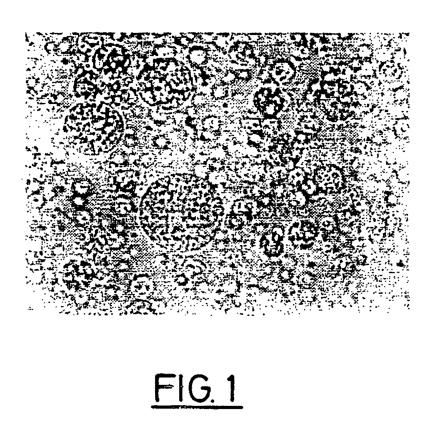

Dans cet essai on prépare une émulsion multiple en dispersant l'émulsion inverse dans une phase aqueuse continue contenant l'agent de surface SDS, à une concentration égale au dixième de sa concentration micellaire critique. La phase inverse est la même que celle décrite précédemment en exemple 1 : la fraction volumique en gouttes d'eau y est présente à une concentration de 10 % en volume avec 0,1 mole/l de NaCl, pour une concentration en agent de surface de 2 %. L'émulsion multiple est donc composée de 90 % en volume d'une phase aqueuse externe et de 10 % de la phase inverse. On applique un taux de cisaillement élevé de l'ordre de 10 000 tours/minute à l'aide d'un dispositif Ultra-turrax pendant 10 secondes à un volume total de 50 m³ de la composition globale, ce qui conduit à l'apparition de gouttes doubles dans lesquelles la teneur en eau et la taille des gouttelettes sont conservées. L'aspect visuel microscopique de cette émulsion multiple, après deux mois de stockage à température ambiante, est représenté en figure 1.

On observe des gouttes doubles, avec chacune d'entre elles contenant des petites gouttes d'eau inverses. Aucun phénomène de coalescence entre les globules doubles larges, n'est observé à cette concentration de SDS après deux mois de stockage.

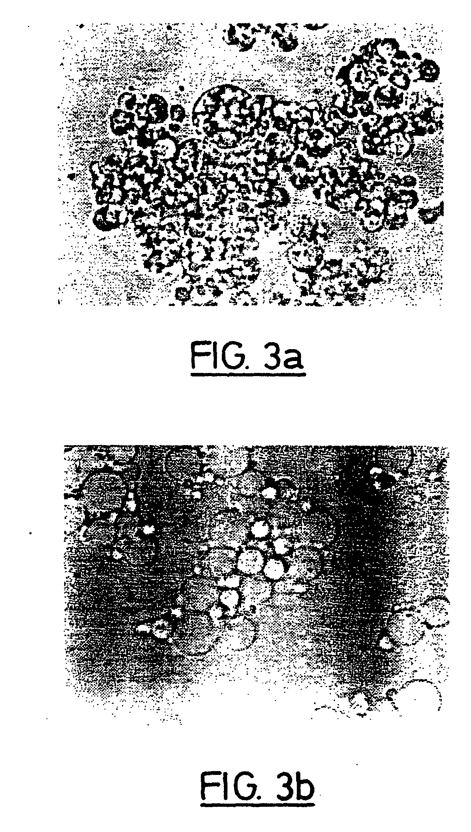

On répète cet essai, en faisant varier la concentration de l'agent de surface à l'intérieur de la phase

aqueuse externe $C_e$, la double émulsion étant fraîchement préparée, et on note la durée d'existence des gouttelettes d'eau internes. On observe ainsi que la double émulsion se conserve (plus d'un mois) ou se transforme en une émulsion directe (dans un délai de quelques heures) selon la concentration en SDS. Cette transition s'opère pour exactement la même concentration que celle mentionnée en exemple 1 c'est-à-dire environ 10 CMC. La figure 3(a) représente la double émulsion juste après sa préparation pour une concentration en SDS de 10 CMC et la figure 3(b) montre la même émulsion après 2 jours. On observe ainsi sous microscope, que seuls des phénomènes de coalescence entre les minuscules gouttes d'eau inverses et l'interface des gouttes directes sont responsables de l'évolution qui est observée.

Par ailleurs, on ne note, dans le laps de temps de cet essai, aucun phénomène de coalescence des gouttelettes inverses à l'intérieur du globule double. On observe uniquement une diminution progressive de la concentration en gouttelettes internes. De ces observations, il ressort donc qu'il n'existe aucun phénomène de coalescence entre ces gouttes internes et que les seuls phénomènes de coalescence existant sont ceux existant entre les minuscules gouttes et l'interface des globules.

**EXEMPLE 4**

Préparation d'une émulsion multiple stabilisée par un agent de surface d'HLB égale à 15, présent dans la phase aqueuse A2.

On prépare des émulsions multiples selon le protocole décrit dans les exemples précédents en utilisant, dans la phase aqueuse A2, à titre d'agent de surface, le Tween 80. Son HLB est de 15 et sa concentration micellaire critique de $10^{-3}$ moles/l. Le même type de résultat est observé. Le transfert des gouttelettes d'eau interne est également tributaire de la concentration $C_e$ de l'agent de surface dans la phase externe. Dans ce cas, la concentration limite est d'environ 200 CMC.

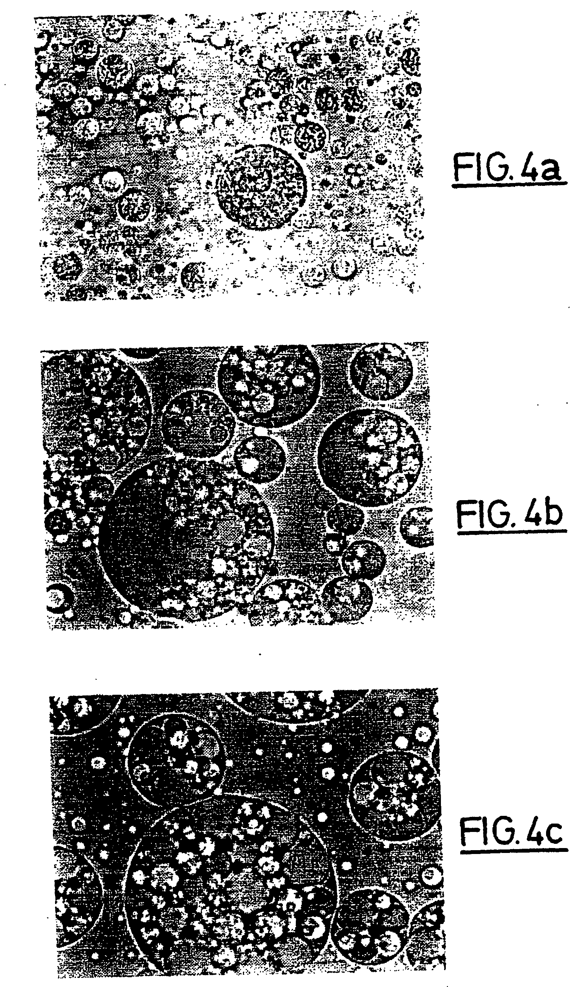

Les figures 4a, 4b et 4c sont des photographies microscopiques de cette double émulsion avec une concentration $C_i$ de 100 CMC et une concentration Ce de CMC/10 respectivement fraîchement préparées (4a), après 5 heures (4b) et après 13 jours (4c).

On observe pour cet agent de surface, que les gouttelettes d'eau internes coalescent entre elles. Cette instabilité apparaît rapidement et conduit à quelques gouttes d'eau interne de surface plus importante, toujours prisonnière des globules huileux.

**EXEMPLE 5**

Détermination de l'effet de la concentration en agent de surface de la phase aqueuse A2 sur la valeur du seuil de concentration.

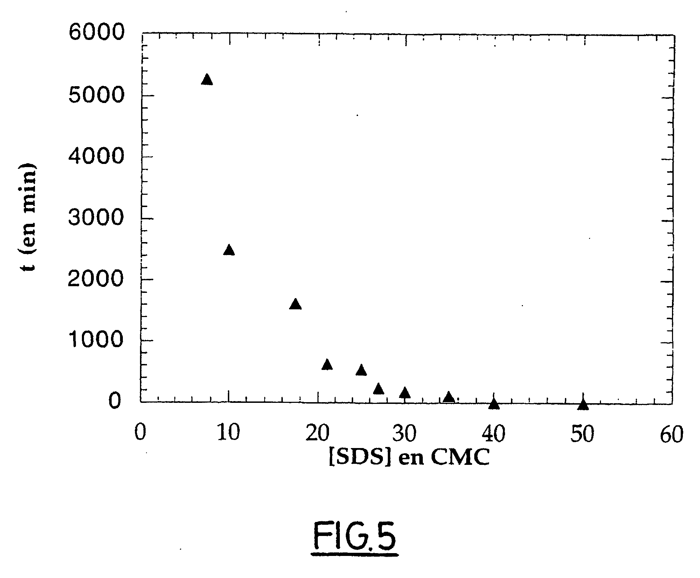

On prépare différentes émulsions selon le protocole défini dans l'exemple n° 1. On fait varier la concentration du tensioactif (SDS) présent dans la phase aqueuse externe et on observe au microscope optique à contraste de phase (Zeiss Axiovert 100) l'évolution temporelle de l'émulsion double. La courbe de la figure 5 rend compte de l'évolution du temps caractéristique de relargage en fonction de la concentration de tensioactif présent dans la phase aqueuse externe. Le temps caractéristique de relargage est défini comme le temps au bout duquel la quasi-totalité des gouttelettes de phase A1 a été relarguée dans la phase aqueuse externe A2 (par coalescence). Comme indiqué, ce temps est apprécié de façon visuelle à l'aide d'un microscope. On remarque que le temps de relargage passe de plusieurs jours lorsque la concentration est inférieure ou égale à 10 CMC à seulement quelques heures quand la concentration est égale à 40 CMC.

**Revendications**

1. Procédé pour libérer de manière contrôlée un principe actif contenu dans une émulsion multiple de type eau dans huile dans eau, ladite émulsion multiple comprenant une émulsion inverse Ei avec une phase aqueuse A1 qui contient au moins un principe actif hydrophile, ladite émulsion Ei étant dispersée sous forme de gouttelettes d'émulsion directe Ed, dans une phase continue aqueuse A2, avec les deux émulsions Ed et Ei stabilisées par au moins un agent de surface, présent au niveau de leurs phases continues respectives, l'agent de surface contenu dans la phase aqueuse A2 de l'émulsion Ed étant un agent de surface hydrosoluble possédant une HLB supérieure à 14, présent à une concentration inférieure à la concentration dite concentration-seuil critique, au-delà de laquelle on induit une déstabilisation de l'émulsion multiple, **caractérisé en ce que** ladite émulsion multiple est mise en présence d'une quantité suffisante d'un agent de surface pour que la concentration en agent de surface dans la phase A2 devienne supérieure à la concentration-seuil critique, de manière à transformer l'émulsion multiple en une émulsion directe et à induire la libération du principe actif, contenu dans la phase aqueuse A1 de l'émulsion Ei, dans la phase aqueuse A2.

2. Procédé selon la revendication 1, **caractérisé en ce que** l'agent de surface ajouté à l'émulsion multiple est un agent de surface identique à celui présent dans la phase aqueuse A2 de l'émulsion Ed.

**3.** Procédé selon l'une des revendications précédentes **caractérisé en ce que** l'agent de surface présent dans la phase continue aqueuse A2 de l'émulsion Ed est choisi parmi les lécithines hydrosolubles, esters de sucrose, esters d'acides gras, alkylamides polyoxyéthylénés, triglycérides sulfates, alkyles sulfates, alkyles éther sulfates, alkyles sulfonates, sels d'alkylamines, amines grasses, lipoamino-acides, alkylbétaines, alkylpolyglycol éthers, copolymères d'oxydes d'alkylènes, polyesters modifiés, tensioactifs polymériques siliconés.

**4.** Procédé selon l'une des revendications précédentes **caractérisé en ce que** l'agent de surface présent dans la phase continue de l'émulsion Ei possède une HLB inférieure à 7.

**5.** Procédé selon l'une des revendications précédentes **caractérisé en ce que** l'agent de surface présent dans la phase continue de l'émulsion Ei est choisi parmi les lécithines liposolubles, les esters de sorbitanne et d'acides gras, polyalkylènes dipolyhydroxystéarates, acides gras, monoglycérides, esters de polyglycérol, polyricinoléate de polyglycérol, esters d'acide lactique et tartrique.

**6.** Procédé selon l'une des revendications précédentes **caractérisé en ce que** la phase contenue de l'émulsion Ei est une phase huileuse composée d'au moins une huile choisie parmi les huiles minérales, végétales ou animales.

**7.** Procédé selon l'une des revendications précédentes **caractérisé en ce que** le principe actif en solution dans la phase aqueuse A1 est un composé choisi parmi les vitamines, enzymes, insuline, les agents antalgiques, antimitotiques, anti-inflammatoires ou anti-glaucomateux, vaccins, agents anticancéreux, antagonistes narcotiques, agents de détoxication, agents dépilatoires, agents correcteurs ou masqueurs de goût, sels hydrosolubles, *agents rupteurs,* acides, bases, vinaigre, glucose, colorants, conservateurs ou leurs mélanges.

**8.** Procédé selon l'une des revendications précédentes **caractérisé en ce que** l'émulsion directe Ed, comprend en volume de 50 à 99 % d'une phase continue aqueuse A2 pour 1 à 50% d'émulsion inverse Ei.

**9.** Procédé selon l'une des revendications précédentes **caractérisé en ce que** l'émulsion inverse Ei est composée en volume de 50 à 99% d'une phase continue pour 1 à 50% de phase aqueuse $A_1$.

**10.** Application d'un procédé selon l'une des revendications 1 à 9 pour le relargage contrôlé d'un agent rupteur dans le cadre de l'application d'un revêtement à base de bitume.

**Claims**

**1.** Process for the controlled release of an active substance contained in a multiple water-in-oil-in-water emulsion, said multiple emulsion comprising an inverse emulsion Ei with an aqueous phase A1 which contains at least one hydrophilic active substance, said emulsion Ei being dispersed in the form of droplets of direct emulsion Ed, in a continuous aqueous phase A2, with the two emulsions Ed and Ei stabilised by at least one surface-active agent, present in their respective continuous phases, the surface-active agent contained in the aqueous phase A2 of the emulsion Ed being a water-soluble surface-active agent having an HLB greater than 14, present in a concentration below the concentration known as the critical threshold concentration, beyond which destabilisation of the multiple emulsion is induced,
**characterised in that** the multiple emulsion is brought into contact with a quantity of surface-active agent such that the concentration of surface-active agent in the phase A2 becomes greater than the critical threshold concentration, so as to convert the multiple emulsion into a direct emulsion and cause the release of the active substance contained in the aqueous phase A1 of the emulsion Ei, into the aqueous phase A2.

**2.** Process according to claim 1, **characterised in that** the surface-active agent added to the multiple emulsion is a surface-active agent identical to that which is present in the aqueous phase A2 of the emulsion Ed.

**3.** Process according to one of the preceding claims, **characterised in that** the surface-active agent present in the continuous aqueous phase A2 of the emulsion Ed is selected from among the water-soluble lecithins, sucrose esters, fatty acid esters, polyoxyethylene alkylamides, triglyceride sulphates, alkyl sulphates, alkyl ether sulphates, alkyl sulphonates, alkylamine salts, fatty amines, lipoamino acids, alkylbetaines, alkyl polyglycol ethers, alkylene oxide copolymers, modified polyesters and polymeric silicon surfactants.

**4.** Process according to one of the preceding claims, **characterised in that** the surface-active agent present in the continuous phase of the emulsion Ei *has* an HLB *of* less than 7.

**5.** Process according to one of the preceding claims, **characterised in that** the surface-active agent present in the continuous phase of the emulsion Ei is selected from among the lipo-soluble lecithins, esters of sorbitan and fatty acids, polyalkylene dipolyhydroxystearates, fatty acids, monoglycerides, polyglycerol esters, polyglycerol polyricinoleate, esters of lactic and tartaric acid.

6. Process according to one of the preceding claims, **characterised in that** the continuous phase of the emulsion Ei is an oily phase made up of at least one oil selected from among the mineral, vegetable or animal oils.

7. Process according to one of the preceding claims, **characterised in that** the active substance dissolved in the aqueous phase A1 is a compound selected from among the vitamins, enzymes, insulin, the analgesics, antimitotics, anti-inflammatories or anti-glaucoma agents, vaccines, anti-cancer agents, narcotic antagonists, detoxifying agents, depilatory agents, flavour correcting or masking agents, water-soluble salts, breakdown agents, acids, bases, vinegar, glucose, colourings, preservatives or mixtures thereof.

8. Process according to one of the preceding claims, **characterised in that** the direct emulsion Ed comprises 50 to 99 % by volume of a continuous aqueous phase A2 to 1 to 50% of inverse emulsion Ei.

9. Process according to one of the preceding claims, **characterised in that** the inverse emulsion Ei is made up of 50 to 99 % by volume of a continuous phase to 1 to 50% of aqueous phase A1.

10. Use of a process according to one of claims 1 to 9 for the controlled salting out of a breaking agent within the scope of the application of a bitumen-based covering.

**Patentansprüche**

1. Verfahren zum gesteuerten Freisetzen eines Aktivstoffs, der in einer Mehrfachemulsion der Art "Wasser in Öl in Wasser" enthalten ist, wobei die Mehrfachemulsion eine inverse Emulsion Ei mit einer wässrigen Phase A1 aufweist, die wenigstens einen hydrophilen Aktivstoff enthält, und die Mehrfachemulsion in Form von Tröpfchen einer direkten Emulsion Ed in einer stetigen wässrigen Phase A2 dispergiert ist, wobei die beiden Emulsionen Ed und Ei durch wenigstens einen grenzflächenaktiven Stoff stabilisiert sind, der in ihrer jeweiligen stetigen Phase vorhanden ist, wobei der grenzflächenaktive Stoff, der in der wässrigen Phase A2 der Emulsion Ed enthalten ist, ein wasserlöslicher grenzflächenaktiver Stoff ist, der einen über 14 liegenden HLB-Wert besitzt und in einer Konzentration vorliegt, die unter der kritischen Konzentrationsschwelle liegt, oberhalb der eine Destabilisierung der Mehrfachemulsion ausgelöst wird,

   **dadurch gekennzeichnet, dass** die Mehrfachemulsion mit einer Menge von grenzflächenaktivem Stoff versehen wird, die dazu ausreicht, dass die Konzentration an grenzflächenaktivem Stoff in der Phase A2 größer als die kritische Konzentrationsschwelle wird, so dass die Mehrfachemulsion in eine direkte Emulsion umgewandelt wird und die Freisetzung des in der wässrigen Phase A1 der Emulsion Ei enthaltenen Aktivstoffs in die wässrige Phase A2 ausgelöst wird.

2. Verfahren nach Anspruch 1, **dadurch gekennzeichnet, dass** der grenzflächenaktive Stoff, der der Mehrfachemulsion beigefügt wird, ein grenzflächenaktiver Stoff ist, der identisch zu demjenigen ist, welcher in der wässrigen Phase A2 der Emulsion Ed vorhanden ist.

3. Verfahren nach einem der vorhergehenden Ansprüche, **dadurch gekennzeichnet, dass** der grenzflächenaktive Stoff, der in der stetigen wässrigen Phase A2 der Emulsion Ed vorhanden ist, aus wasserlöslichen Lecithinen, Sucroseestern, Fettsäureestern, Polyoxyethylenalkylamiden, Triglyceridsulfaten, Alkylsulfaten, Alkyläthersulfaten, Alkylsulfonaten, Alkylaminsalzen, Fettaminen, Fettaminosäuren, Alkylbetainen, Alkylpolyglycoläthern, Alkylenoxid-Copolymeren, modifizierten Polyestern, polymerischen Silikon-Tensiden gewählt wird.

4. Verfahren nach einem der vorhergehenden Ansprüche, **dadurch gekennzeichnet, dass** der grenzflächenaktive Stoff, der in der stetigen Phase der Emulsion Ei vorhanden ist, einen HLB-Wert von weniger als 7 besitzt.

5. Verfahren nach einem der vorhergehenden Ansprüche, **dadurch gekennzeichnet, dass** der grenzflächenaktive Stoff, der in der stetigen Phase der Emulsion Ei vorhanden ist, aus fettlöslichen Lecithinen, Sorbitan- und Fettsäureestern, Dipolyhydroxystereat-Polyalkylenen, Fettsäuren, Monoglyceriden, Polyglycerolestern, Polyglycerol-Polyricinoleat, Milch- und Weinsäureestern gewählt wird.

6. Verfahren nach einem der vorhergehenden Ansprüche, **dadurch gekennzeichnet, dass** die stetige Phase der Emulsion Ei eine Ölphase ist, die aus wenigstens einem Öl zusammengesetzt ist, das aus Mineral-, Pflanzen- oder Tierölen gewählt wird.

7. Verfahren nach einem der vorhergehenden Ansprüche, **dadurch gekennzeichnet, dass** der in der wässrigen Phase A1 gelöste Aktivstoff eine Zusammensetzung ist, die aus Vitaminen, Enzymen, Insulin, Schmerzmitteln, Zellteilungshemmern, Entzündungshemmern oder Glaukomgegenmitteln, Impfstoffen, Tumorgegenmitteln, Betäubungsgegenmitteln, Entgiftungsmitteln, Enthaarungsmitteln, Geschmacksveränderungs- oder Geschmacksverdeckungsmitteln, wasserlöslichen Salzen, Aufbruchmitteln, Säuren, Basen, Essig, Glucose, Färbemitteln, Konservierungsstoffen oder deren Mischungen gewählt ist.

8. Verfahren nach einem der vorhergehenden Ansprüche, **dadurch gekennzeichnet, dass** die direkte Emulsion Ed zu 50 bis 99 Volumenprozent eine stetige wässrige Phase A2 auf 1 bis 50% der inversen Emulsion Ei umfasst.

9. Verfahren nach einem der vorhergehenden Ansprüche, **dadurch gekennzeichnet, dass** die inverse Emulsion Ei zu 50 bis 99 Volumenprozent eine stetige Phase auf 1 bis 50% der wässrigen Phase A1 umfasst.

10. Anwendung eines Verfahrens nach einem der Ansprüche 1 bis 9 zur gesteuerten Freisetzung eines Aufbruchmittels im Rahmen der Aufbringung eines auf Bitumen basierenden Belags.

FIG. 1

FIG. 2

FIG. 3a

FIG. 3b

FIG. 4a

FIG. 4b

FIG. 4c

FIG.5